# EUROPEAN PATENT APPLICATION

(11) **EP 3 235 540 A1**
(43) Date of publication of application: **25.10.2017**
(21) Application number: 15868270.8
(22) Date of filing: 07.12.2015
(51) Int. Cl.: A61N 1/36

(54) **AUXILIARY DEVICE FOR TRAINING AND AUXILIARY METHOD FOR TRAINING**

(30) Priority: 09.12.2014 CN 201410749425
(71) Applicant: Beijing Galaxy Raintai Technology Co., Ltd., Beijing 100036 (CN)
(72) Inventor: TANG, Yuxin, Beijing 100036 (CN); YAN, Wenwen, Beijing 100036 (CN)
(74) Representative: dompatent von Kreisler Selting Werner - Partnerschaft von Patent- und Rechtsanwälten mbB
(86) International application number: PCT/CN2015/096532
(87) International publication number: WO 2016/091130

(57) **Abstract**

The present invention relates to a training assistance apparatus and a training assistance method. The training assistance apparatus comprises a collecting unit, a main control unit and a discharging unit; the collecting unit is configured to collect an electromyographic signal of a user's specific position during implementation of actions and output it to the main control unit after amplification and filtering; the main control unit is configured to determine an optimal electromyographic signal according to the received electromyogaphic signal, use the optimal electromyoelectrical signal to form a stimulation signal, output the stimulation signal to the discharging unit; the discharging unit forms a stimulation current according to the stimulation signal and applies the stimulation current upon the user's specific position. The present invention employs a closed-loop electromyographic stimulation manner of performing equivalence between the "collection-stimulation" type electromyographic signal and stimulation signal, and enables the action of a trainee to develop in an optimal direction. The progressive manner of the present invention more facilitates improving the training effect as compared with a fixed programmed manner in the prior art. In addition, the present invention does not limit constraints of place and time.

## Description

This application claims priority to the Chinese patent application No.201410749425.2 entitled "Training Assistance Apparatus and Training Assistance Method" filed on December 9, 2014, the entire disclosure of which is hereby incorporated by reference in its entirety.

### Field of the Invention

The present invention relates to the technical field of computer application, and particularly to a training assistance apparatus and a training assistance method.

### Background of the Invention

As people's material and cultural life develops rapidly, training markets of for example art and physical education have a huge development space and broad prospect. Take the field of musical instrument training as an example. At present, a mostly-employed training manner is face-to-face training, namely, a parent chooses a music teacher to teach the kid to learn a musical instrument. However, in this manner, on the one hand the parent lacks judgment criteria for selecting a teaching as lacking expertise, and cannot evaluate the student's training effect. The teacher usually performs one-to-one training with a lower teaching efficiency. Furthermore, this training manner causes high training costs to the parents and is usually constrained by regions and time.

To cut training costs and improve education efficiency and break away from constraints of regions and time, an audio and video teaching manner arises, namely, students learn courses presented in audio and video manners by virtue of the Internet or resources locally stored in the computer. However, this manner mainly relies on self-study and results in an undesirable training effect.

To solve the above problems, in the prior art some people propose an electrical stimulation type training device, namely, an electrical stimulation training program is preset in a processing module by computer programming, the processing module directly controls a digital-analog converter (DAC) according to the program to transmit an analog signal to a constant-current source, then the constant-current source generates a constant current, a plurality of constant-current sources are connected to a plurality of electrodes, and these electrodes respectively perform electrical stimulation for a finger or arm. A user wearing this device can, being electrically stimulated, passively make continuous action on the musical instrument. In this way, this device achieves a purpose of guiding the user to play the musical instrument automatically in the form. However, this manner has the following drawbacks:
1) This manner employs a fixed program and does not specifically distinguish users; no matter whether the users are adult or kids and regardless of the users' playing skills, they employ the same stimulation signal and this manner does not have a progressive function. Hence, the training effect is undesirable.
2) The electrical stimulus employed is of at least tens of mA; large-current stimulation in a long period of time might cause potential hurt to skin tissue, and this manner is not adapted for users such as children.

### Summary of the Invention

In view of this, the present invention provides a training assistance apparatus and a training assistance method to refrain from constraints of place and time and improve a training effect.

A specific technical solution is as follows:
The present invention provides a training assistance apparatus, comprising a collecting unit, a main control unit and a discharging unit;
the collecting unit is configured to collect an electromyographic signal of a user's specific position during implementation of actions and output it to the main control unit after amplification and filtering;
the main control unit is configured to determine an optimal electromyographic signal according to the received electromyogaphic signal, use the optimal electromyoelectrical signal to form a stimulation signal, output the stimulation signal to the discharging unit;
the discharging unit forms a stimulation current according to the stimulation signal and applies the stimulation current upon the user's specific position.

According to a preferred embodiment of the present invention, the collecting unit comprises a detection electrode, an amplifying module and a filtering module;
the detection electrode is disposed at the user's specific position and used to collect the electromyographic signal at the user's specific position, and output it to the amplifying module;
the amplifying module is configured to perform amplifying processing for the electromyoelectrical signal and then output it to the filtering module;
the filtering module is configured to perform filtering processing for the electromyographic signal output by the amplifying module, and then output to the main control unit.

According to a preferred embodiment of the present invention, the main control unit comprises an analog-digital conversion module, a processing module and a digital-analog conversion module;
the analog-digital conversion module is configured to perform analog-digital conversion for the electromyographic signal output by the collecting unit to obtain a digital electromyographic signal and then output it to the processing module;
the processing module is configured to use the received digital electromyographic signal to determine an optimal electromyographic signal, use the optimal electromyogrphic signal to equivalently work out a simulation signal and output the stimulation signal to the digital-analog conversion module;
the digital-analog conversion module is configured to perform digital-analog conversion for the received stimulation signal and then output it to the discharging unit.

According to a preferred embodiment of the present invention, the training assistance apparatus further comprises: a communication unit;

Upon determining the optimal electromyographic signal, the processing module specifically executes the following: send the collected electromyographic signal to a terminal equipment via the communication unit, and obtain the stimulation signal returned by the terminal equipment via the communication unit, the stimulation signal being equivalently worked out using the optimal electromyographic signal after the terminal equipment analyzes and compares the collected electromyograhic signal to determine the optimal electromyographic signal; or, analyze and compare the collected electromyographic signal to determine the optimal electromyographic signal, and send the optimal electromyograpic signal to the terminal equipment via the communication unit, obtain the stimulation signal returned by the terminal equipment via the communication unit, the stimulation signal being equivalently worked out by the terminal equipment using the optimal electromyographic signal.

According to a preferred embodiment of the present invention, upon determining the optimal electromyographic signal, the processing module specifically executes the following: analyze the received digital electromyographic signal to obtain various indices of the action of this time, compare the electromyographic signal of the action of this time with the electromyographic signals of actions in the past in respect of various indices, to determine the optimal electromyograhic signal therein.

According to a preferred embodiment of the present invention, using the optimal electromyographic signal to equivalently work out the stimulation signal, the processing module specifically executes determining a cyclic signal matching with the optimal electromyographic signal in power, and amplify the cyclic signal as the stimulation signal.

According to a preferred embodiment of the present invention, a frequency of the cyclic signal is over twice a frequency of the electromyographic signal.

According to a preferred embodiment of the present invention, an amplifying multiple employed by the processing module upon amplifying the cyclic signal satisfies V*G<R*I, wherein the V is a maximum intensity of the optimal electromyographic signal, R is a human body equivalent resistance, and I is a stimulation current intensity that can be received by the human body without pains.

According to a preferred embodiment of the present invention, the training assistance apparatus further comprises a nine-axis sensor;
the nine-axis sensor is used to collect the user's movement data and output it to the processing module;
the processing module is further used to combine with the movement data to determine the optimal electromyographic signal.

According to a preferred embodiment of the present invention, the discharging unit comprises a forward side summator, a voltage-controlled constant-current source and a discharging electrode;
the forward side summator is configured to convert the stimulation signal output by the main control unit into a bipolar signal and then output the bipolar signal to the voltage-controlled constant-current source;
the voltage-controlled constant-current source is configured to output a corresponding stimulation current according to the input bipolar signal;
the discharging electrode is used to enable the stimulation current output by the voltage-controlled constant-current source to act upon the user's specific position.

According to a preferred embodiment of the present invention, the collecting unit and the discharging unit respectively have N channels, wherein the N is a positive integer greater than 1, and the channel of the collecting unit corresponds one-to-one with the channel of the discharging unit;
the discharging unit further comprises: a multi-path selection module controlled by the main control unit to select a channel, and send the bipolar signal output by the forward side summator to the corresponding voltage-controlled constant-current source module of the corresponding channel in turn at divided time.

According to a preferred embodiment of the present invention, the detection electrode in the collecting unit and the discharging electrode in the discharging unit share a differential electrode in a frequency division manner.

The present invention further provides a training assistance method, comprising:
collecting an electromyographic signal of a user's specific position during implementation of actions and performing amplification and filtering for the electromyographic signal;
determining an optimal electromyographic signal according to the electromyogaphic signal after amplification and filtering, and using the optimal electromyoelectrical signal to form a stimulation signal;
forming a stimulation current according to the stimulation signal and applying the stimulation current upon the user's specific position.

According to a preferred embodiment of the present invention, the determining an optimal electromyographic signal according to the electromyogaphic signal after amplification and filtering, and using the optimal electromyoelectrical signal to form a stimulation signal comprises:
performing analog-digital conversion for the amplified and filtered electromyographic signal to obtain a digital electromyographic signal;
analyzing and comparing the digital electromyographic signal to determine an optimal electromyographic signal;
using the optimal electromyograhic signal to equivalently work out the stimulation signal;
performing digital-analog conversion for the stimulation signal.

According to a preferred embodiment of the present invention, the analyzing and comparing the digital electromyographic signal to determine an optimal electromyographic signal comprises:
analyzing the digital electromyographic signal to obtain various indices of the action of this time;
comparing the electromyographic signal of the action of this time with the electromyographic signals of actions in the past in respect of various indices, to determine the optimal electromyograhic signal.

According to a preferred embodiment of the present invention, the using the optimal electromyograhic signal to equivalently work out the stimulation signal comprises:
determining a cyclic signal matching with the optimal electromyographic signal in power, and amplifying the cyclic signal as the stimulation signal.

According to a preferred embodiment of the present invention, a frequency of the cyclic signal is over twice a frequency of the electromyographic signal.

According to a preferred embodiment of the present invention, an amplifying multiple employed upon amplifying the cyclic signal satisfies V*G<R*I, wherein the V is a maximum intensity of the optimal electromyographic signal, R is a human body equivalent resistance, and I is a stimulation current intensity that can be received by the human body without pains.

According to a preferred embodiment of the present invention, the method further comprises: using a nine-axis sensor to collect the user's movement data, and further combining the movement data upon determining the optimal electromyographic signal.

According to a preferred embodiment of the present invention, the forming a stimulation current according to the stimulation signal comprises:
converting the stimulation signal into a bipolar signal;
using the bipolar signal as an input signal of the voltage-controlled constant-current source to obtain the stimulation current output by the voltage-controlled constant-current source.

As can be seen from the above technical solution, the present invention employs a closed-loop electromyographic stimulation manner of performing equivalence between the "collection-stimulation" type electromyographic signal and stimulation signal, and enables the action of the trainee to develop in an optimal direction by performing stimulation based on the optimal electromyographic signal during implementation of actions of the specific trainee. The progressive manner of the present invention more facilitates improving the training effect as compared with a fixed programmed manner in the prior art. In addition, the present invention does not limit constraints of place and time.

### Brief Description of Drawings

Fig. 1 is a structural view of a training assistance apparatus according to an embodiment of the present invention;
Fig. 2 is a schematic diagram showing cyclical relationship of an electromyographic signal and a stimulation signal according to an embodiment of the present invention;
Fig. 3 is a preferred structural schematic view of a training assistance apparatus according to an embodiment of the present invention;
Fig. 4 is a structural schematic diagram of an electrode plate of a training assistance apparatus according to an embodiment of the present invention;
Fig. 5 is a diagram of a preferred example of a training assistance apparatus according to an embodiment of the present invention;
Fig. 6 is a flow chart of a training assistance method according to an embodiment of the present invention;
Fig. 7 is a flow chart of a method of forming a stimulation signal according to an embodiment of the present invention.

### Detailed Description of Preferred Embodiments

Embodiments of the present invention will be further described in detail with reference to figures to make objects, technical solutions and advantages of the present invention clearer.

A kernel idea of the present invention lies in collecting an electromyographic signal of a user's specific position during implementation of actions, and collecting an equivalent stimulation type closed electromyographic stimulation, i.e., using the collected electromyographic signal during implementation of actions to determine an optimal electromyographic signal, the optimal electromyographic signal forming a stimulation signal to action upon the user's specific position, thereby achieving an effect of enhancing muscle memory and enabling a trainee's playing to develop in an optimal direction.

To facilitate understanding, brief introduction is first given to electromyographic signal. The electromyographic signal is a temporal and spatial comprehensive result of a complicated hypodermic muscle activity at the skin surface. The electromyographic signal originates from motoneuron in spinal cord, a cell body axon of the motoneuron extends to the fibers, and is coupled to muscle fibers via an end plate region.

The electromyographic signal has the following characteristics: the electromyographic signal at the human body surface is very weak and ranges from several microvolt to several millivolt. The electromyographic signal has an alternating property and is an out-of-order AC voltage signal, it is substantially proportional to a muscular tension generated when the muscle impulses, and the electromyographic signal collected under different intensities also varies. When the muscle changes constantly, a mean square value of the electromyographic signal is time-variant, the signal is unstable and may be understood as amplitude modulation noise. A spectral range of the electromyographic signal at the human body skin surface is 0-1000Hz, a maximum frequency of power spectrum depends on the muscle and usually ranges from 10 to 200Hz, main energy of the electromyographic signal concentrates below 200Hz, the electromyographic signal has an obvious low-frequency property, and has a fixed frequency band.

A physiological basis of the present invention is that a specific joint movement of a limb is controlled by a corresponding muscle group, the electromyographic signal of the skin surface can not only reflect a joint flexion degree, but also obtain a shape, position and movement information of a gesture in real time. At present, there is already a document showing that the manner of employing the electromyographic signal collection may distinguish tens of gesture actions with a correctness rate of higher than 90%. Take musical instrument playing as an example. The playing procedure substantially includes continuous arm and finger movements, and electromyographic signal collection may well record a complete playing procedure. The electromyographic signal collection may help the user to record playing information to perform basic fingering training and hand shape training, and meanwhile cover factors such as rhythm and continuity. Since the electromyographic signal is an electrical signal originating from the human body and characterized by directness and naturalness, and detecting the electromyographic signal from the skin at the surface of the muscle is a safe electromyographic signal extraction manner.

The human body muscle has a memory effect. After the same action is repeated for many times, the muscle will form an acquired reflex. The human body muscle obtains memory at a very slow speed, but once the memory is obtained, a forgetting speed is very slow. Musical instrument learners mostly feel that fingers needn't be commanded by brain after fingers gets skilled after a lot of practice. When meeting a certain chord or a certain key, the finger will naturally poses a chord shape or naturally moves to beside the piano key. Fingers seem to move by themselves. In contrast, it takes a lot of time to memorize the chord position and the musical score. This indicates that people's brain does not remember these, and instead people's long-term repetition causes muscles at specific positions to generate memory.

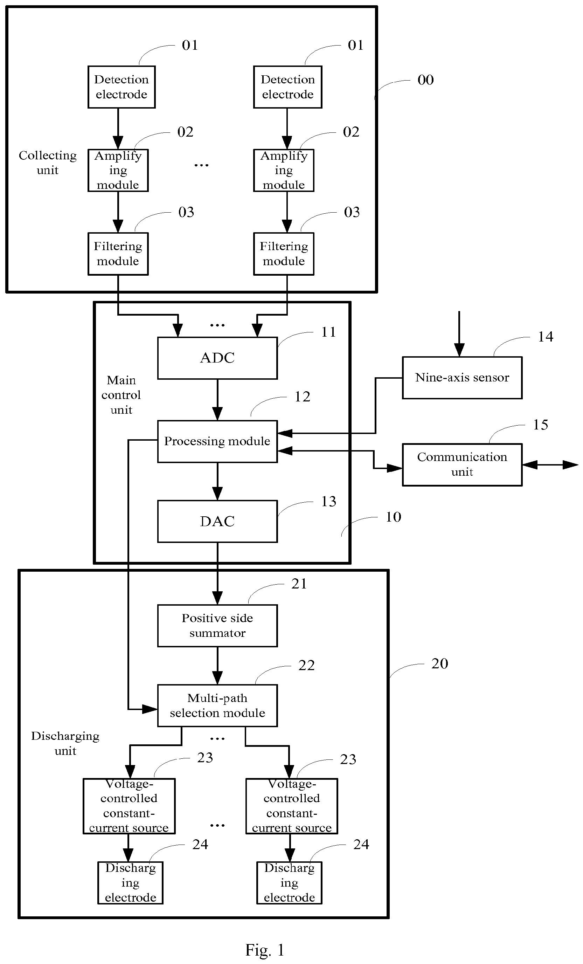

On the above theoretical basis, the training assistance apparatus according to the present invention may be shown in Fig. 1, and mainly comprises three portions: a collecting unit 00, a main control unit 10 and a discharging unit 20, and may comprise a nine-axis sensor 30 and a communication unit 40.

The collecting unit 00 is configured to collect an electromyographic signal of a user's specific position during implementation of actions and output it to the main control unit 00 after amplification and filtering. The main control unit 10 is configured to determine an optimal electromyographic signal according to the electromyogaphic signal output by the collecting unit 00, use the optimal electromyoelectrical signal to form a stimulation signal, output the stimulation signal to the discharging unit 20, and the discharging unit 20 forms a stimulation current according to the stimulation signal to act upon the user's specific position.

The collecting unit 00 may specifically include: a detection electrode 01, an amplifying module 02 and a filtering module 03. The detection electrode 01 contacts the skin, and is disposed at the user's specific position. The specific position is determined according to a specific training program. The detection electrode 01 may be more than one detection electrode. For example, if the training is piano training, the detection electrode 01 may be disposed to multiple fingers; if the training is ball training, the detection electrode 01 is disposed at a finger, wrist and arm. During action implementation, the user makes continuous actions, and the electrode 01 can collect the electromyographic signal at the user's specific position. For example, when the user plays a piano musical composition, the detection electrode 01 can collect the electromyographic signal of the user's fingers.

The amplifying module 02 is configured to perform amplifying processing for the electromyoelectrical signal collected by the detection electrode 01. Since the electromyographic signal at the human body surface is very weak and ranges from several microvolt to several millivolt, it needs to be amplified into a measurable and processable signal. The amplification multiple of the amplifying module 02 may be determined according to the processing capacity and precision of the main control unit 10.

The filtering module 03 is configured to perform filtering processing for the electromyographic signal processed by the amplifying module 02, and then output the electromyographic signal after filtering processing to the main control unit 10. Since the electromyographic signal is collected by the detection electrode 01 disposed at the skin surface, the collection procedure is apt to influence from the surrounding environment, e.g., intrinsic noise of the electronic instrument, movement noise of detection electrode 01 and skin contact surface, and environment noise caused by electromagnetic radiation. The filtering module 03 filters these noise in a band-pass filtering manner.

Noticeably, one detection electrode 01, amplifying module 02 and filtering module 03 form a channel. The collecting unit 00 may include more than one channel which respectively collect the electromyographic signal at different positions, perform amplifying and filtering and then output the signal to the main control unit 10.

As shown in Fig. 1, the main control unit 10 may include: an analog-digital conversion module (ADC) 11, a processing module 12 and a digital-analog conversion module (DAC) 13, wherein the ADC 11 is configured to perform analog-digital conversion for the electromyographic signal output by the collecting unit 00 to obtain a digital electromyographic signal and then provide it to the processing module 12.

The processing module 12 is configured to determine an optimal electromyographic signal and use the optimal electromyographic signal to form a stimulation signal and provide it to the DAC 13. The processing module 12 stores and analyzes the electromyographic signal from the collecting unit 00 to obtain various indices of the action of this time, for example indices such as correctness, tension, and rhythmical accuracy of the action. The specific indices may depend on a specific training program. The electromyographic signal of the action of this time is compared with the electromyographic signals of actions in the past in respect of various indices, to determine the optimal electromyograhic signal therein and use the optimal electromyographic signal to equivalently work out the stimulation signal. If the determined optimal electromyographic signal does not change, the previously-determined stimulation signal is still employed. If the determined optimal electromyographic signal changes, the new optimal electromyographic signal is used to equivalently work out the stimulation signal to replace the previously-determined stimulation signal.

Take piano-playing as an example. The user controls the playing by moving fingers, and movement of fingers is mainly controlled by the muscle group of a forearm. Hence, the user, during playing, will generate a corresponding electromyographic signal sequence, analyzes the electromyographic signal sequence, and extracts time-domain and/or frequency feature of the signal to perform fine-granularity playing action analysis to obtain various incidences. In a standard playing state, the user's arm is in a loose state, and the electromyographic signal is generated only at the playing instant. The amplitude and duration of the electromyographic signal can be judged through an algorithm, and it is further feasible to infer a force-applying degree and a tension degree when the user plays. When the amplitude is larger or the duration is longer, this indicates that the user is in a tensioned state. The user's finger playing rhythm may be judged by analyzing an interval of peaks occurring in the user's electromyographic signal, and then the rhythm is compared with a standard musical composition rhythm to know accuracy of the user's playing rhythm; it is feasible to, through machine learning algorithm of finger actions, identify the fingers used by the user to press keys, namely, determine the user's finger sequence upon playing, and further analyze correctness of the action. The determination of various indices of actions is only illustratively introduced. How to specifically determine various indices of the action is not major content limited by the present invention.

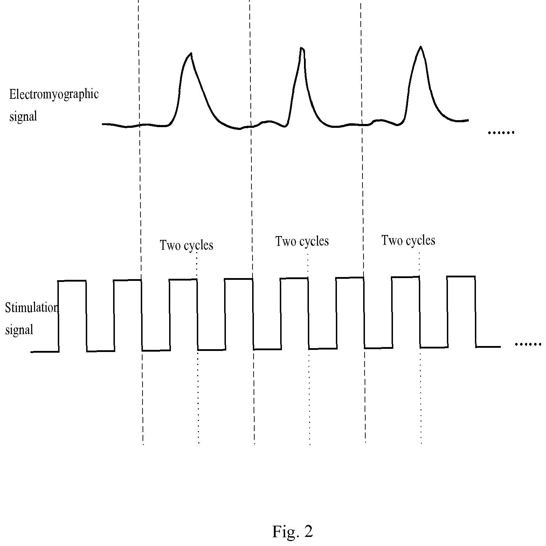

Hereunder, description is given to the procedure of using the optimal electromyographic signal to equivalently work out the stimulation signal. Since the electromyographic signal is a series of statistics results of action potential and it is impossible to restore potential of muscle fibers, an equivalent power method may be employed. Specifically, a power-matched cyclic signal is used to equivalently work out the optimal electromyographic signal, then the power-matched cyclic signal is amplified as the stimulation signal, and the cyclic signal may be sinusoidal wave, square wave or the like. It is feasible to obtain an electromyograhic signal intensity corresponding to each sampling point at a fixed sampling frequency. Since a frequency of the stimulation signal might be inconsistent with the frequency of the electromyographic signal, preferably over twice the electromyographic signal frequency, it is feasible to emit the stimulation signal of more than two cycles at an interval time of each sampling point, as shown in Fig. 2. The power of the stimulation signal is closely related to the amplitude, and the amplitude of the stimulation signal of more than two cycles is equivalently worked out according to the optimal electromyographic signal of each cycle.

In addition, due to fragility of the electromyographic signal, an amplifying multiple is needed after power equivalence, i.e., the stimulation signal needs to be amplified by a fixed multiple. The amplifying multiple here depends on three factors: a stimulation current intensity I that can be received by the human body without pains, a maximum idensity V of the optimal electromyographic signal, and a human body equivalent resistance R. The amplifying multiple G only needs to satisfy the following inequation: V*G<R*I.

Universally, the stimulation current intensity that can be received by the human body without pains is 1mA, the maximum intensity of the collected electromyographic signal is 5mV, and the human body equivalent resistance is 1K Ω, whereupon the amplifying multiple may take 200.

Then, the DAC 13 performs digital-analog conversion for the stimulation signal obtained by the processing module 12 and then outputs it to the discharging unit 20.

In addition, it is appreciated that the processing module 12 may execute the above procedure of determining the optimal electromyographic signal and equivalently working out the stimulation signal. There is a further implementation mode, namely, the processing module 12 sends the collected electromyographic signal to a terminal equipment (e.g., a mobile phone, a flat panel computer, PC or even an integrated circuit) via a communication unit 40, the terminal equipment executes the above procedure of determining the optimal electromyographic signal and equivalently working out the stimulation signal, and then returns the stimulation signal to the communication unit 40, and the communication unit 40 provides it to the processing module 12.

There is a further implementation mode, i.e., the processing module 12 uses the collected electromyographic signal to determine the optimal electromyographic signal, the sends the optimal electromyographic signal to the terminal equipment via the communication unit 40, the terminal equipment executes the procedure of equivalently working out the stimulation signal, then returns the stimulation signal to the communication unit 40, and the communication unit 40 provides it to the processing module 12.

In addition, the nine-axis sensor 30 may collect movement data. The nine-axis sensor 30 comprises a three-axis gyro, a three-axis acceleration sensor and a three-axis magnetic induction sensor. The processing module may transmit the movement data collected by the nine-axis sensor 30 together with the electromyographic signal to the terminal. The movement data collected by the nine-axis sensor 30 may be converted into a movement speed, a movement displacement and a real-time posture within a 3D space, and in combination with the electromyographic signal, can provide necessary dynamic capturing information for the processing module 12 to analyze and determine the optimal electromyoelectrical signal.

As shown in Fig. 1, the discharging unit 20 may specifically comprise a forward side summator 21, a voltage-controlled constant-current source 23 and a discharging electrode 24.

The forward side summator 21 is configured to convert the stimulation signal output by the main control unit 10 into a bipolar signal and then output it to the voltage-controlled constant-current source 23.

The voltage-controlled constant-current source 23 is configured to output corresponding stimulation current according to the input bipolar signal. The stimulation current output by the voltage-controlled constant-current source 23 is irrelevant to a load resistance, is only related to an input voltage, i.e., the output stimulation current is controlled by the voltage of the bipolar signal.

The discharging electrode 24 enables the stimulation current output by the voltage-controlled constant-current source 23 to act upon the skin at the user's specific position.

When the sampling unit 00 includes N channels, the corresponding discharging unit 20 also includes N channels, and correspondingly the main control unit 10 also includes N DAC 13. The sampling unit 00 corresponds one-to-one with the discharging unit 20, and the detection electrode 01 and stimulation electrode 24 of one channel are usually disposed at the same user position. In the discharging unit 20, each channel may include a forward side summator 21, a voltage-controlled constant-current source 23 and a discharging electrode 24.

However, to reduce the number of forward side summators 21 and DAC 13 and reduce costs and the size price, a multi-path selection module 22 may be disposed in the discharging unit 20, whereupon the main control unit 10 only needs one DAC 13, and the discharging unit 20 only needs one forward side summator 21.

The multi-path selection module 22 is controlled by the processing module 12 in the main control unit 10 and selects a channel, and then the bipolar signal is sent to the corresponding voltage-controlled constant-current source module 23 in turn at divided time.

Since the collecting procedure and the discharging procedure are a pair of reversible procedures, the same electrode may be used for collection and discharging, that is to say, the detection electrode and the discharging electrode may share the electrode, i.e., jointly use one differential electrode. Here, the stimulation signal and the electromyographic signal use a differential frequency band so that the collection procedure and the discharging procedure may work at the same time. Preferably, the collection and stimulation may be simultaneously performed for the muscle at the same position, and the user's data may be stored while stimulation is performed. That is to say, the frequency of the stimulation signal is set out of the frequency band of the electromyographic signal. Since the frequency of the electromyographic signal is between 10-200Hz, the frequency of the stimulation signal should be less than 10Hz or greater than 200Hz. Since the stimulation signal during the discharging procedure needs to discharge one time at each sampling point according to the sampling signal (collected electromyographic signal) and meanwhile a cut-off frequency of the filtering portion is considered, the stimulation signal should be selected more than twice a sampling rate of the collected electromyograhic signal. An effective frequency of the electromyogaphic signal itself is below 500Hz and a sampling rate is 1000Hz. If the stimulation signal frequency during discharging is higher than 2000Hz, the electromyograhic signal of the skin is collected while the apparatus discharges. Since the filtering module performs band-pass filtering for the electromyographic signal and the frequency band is selected lower than 1000Hz, the stimulation signal of the apparatus itself may be well filtered and the collected electromyograhic signal will not be mixed into the simulation signal. In this case, the collection of the electromyograhic signal does not conflict with the stimulation signal, and synchronous operation of collection and discharging is achieved.

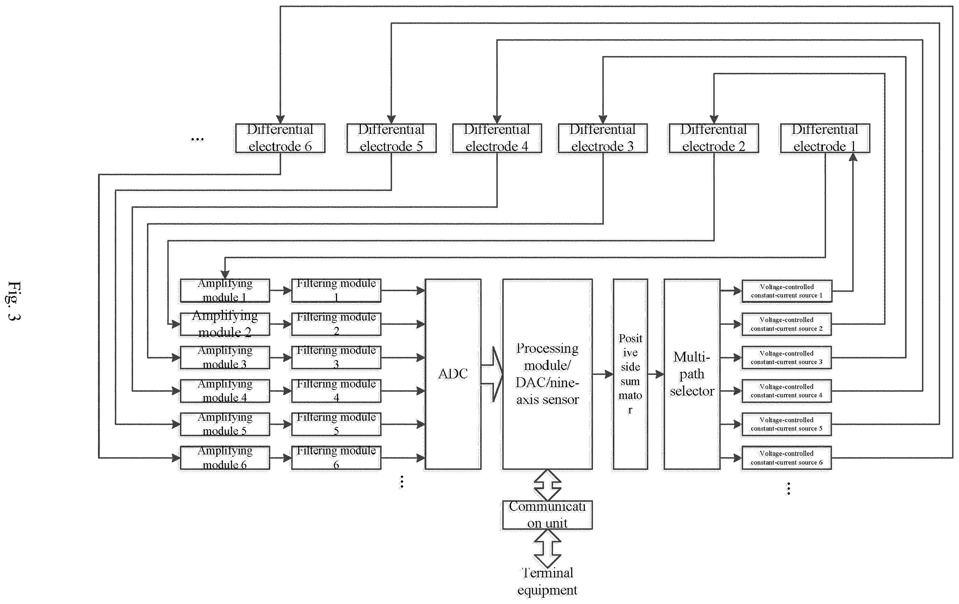

When the detection electrode and the discharging electrode share the differential electrode, a preferred structure of the training assistance apparatus may be as shown in Fig. 3.

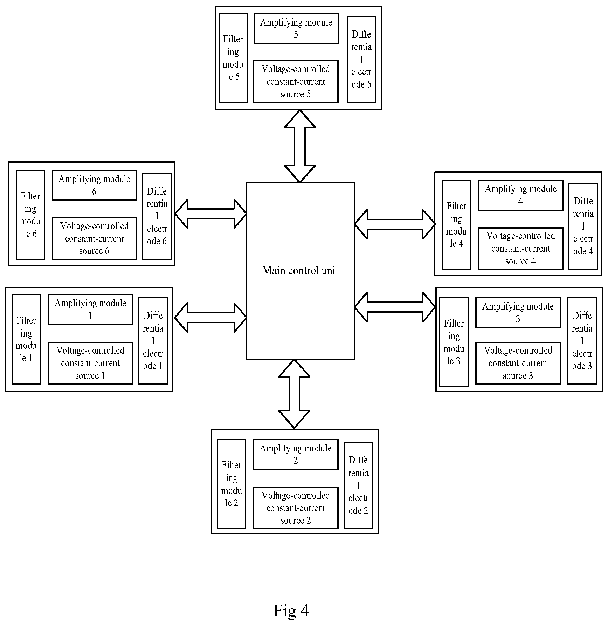

In addition, the structure of the training assistance apparatus may employ a modularized manner. The collecting unit and the corresponding discharging unit may be integrated together, and the detection electrode and the discharging electrode share a differential electrode. That is, the amplifying module, the filtering module, the voltage-controlled constant-current source and the differential electrode are integrated on one electrode plate, the main control unit is integrated on one electrode plate so that the main control unit may communicate with a plurality of electrode plates simultaneously, as shown in Fig. 4.

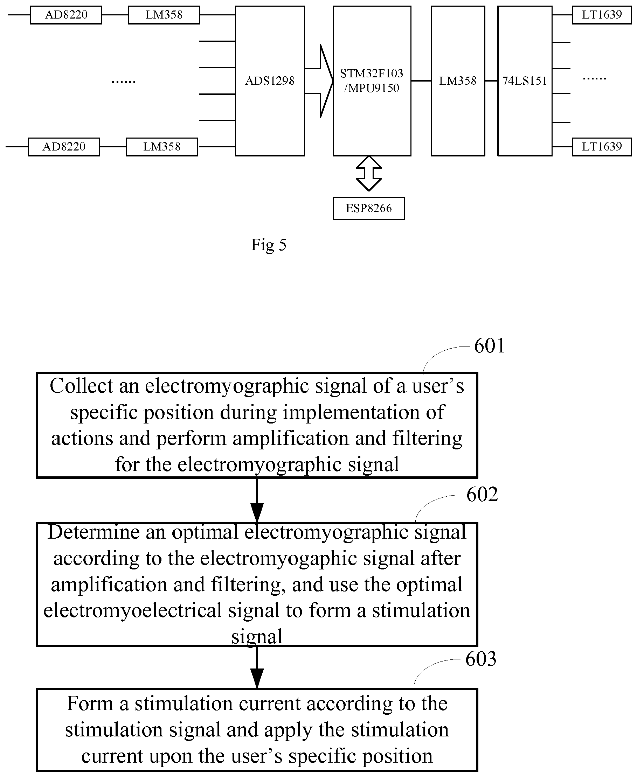

As a preferred embodiment, as shown in Fig. 5, the above amplifying module 02 may employ AD8220 which is an operation amplifier with less noise and has a better common-mode rejection ratio. The filtering module 03 may employ LM358 to achieve two second-order filters, one is a low-pass filter and the other is a high-pass filter, to thereby perform the function of the band-pass filter. The low-pass filter has a resistance of 10.7K, a capacitance of 0.1uf and a cut-off frequency of 400Hz; the high-pass filter has a resistance of 160K, a capacitance of 0.1uf, and a cut-off frequency of 10Hz.

ADC 11 may employ an ADS 1298 analog-digital conversion chip, and it has 8 channels and 24 bits and is adapted for a portable device. In addition, ADS 1298 includes 8 low-noise PGAs (programmable gain amplifiers) and 8 high-resolution synchronous sampling ADCs. Power consumption of each channel is only 1mW. As compared with discrete implementation, its power fall amplitude achieves up to 95% and thereby improves portability of the apparatus.

The processing module 12 may employs a STM32F103 which is a commonly-used enhanced type microcontroller which has a 32-bit Cortex-M3 internal core and whose maximum operation frequency achieves 72MHz. A dual-channel DAC is built in the STM21F103. The DAC 13 in the present invention may be implemented by only using one DAC therein.

The nine-axis sensor 30 may employ MPU-9400 which is the first instance of a nine-axis movement sensing tracking assembly in the world, and comprises a three-axis gyro, a three-axis acceleration sensor and a three-axis induction sensor, and is low-power consumption, low-cost, and high-performance consumptive electronic chip. The MPU-9400 includes InvenSense movement sensing fusion calculus and movement correction.

To reduce operation pressure of a main chip firmware of a processing module, reduce command execution number, and process posture fusion resolution of the three-axis gyro, three-axis acceleration sensor and three-axis magnetic induction sensor by using a DMP (digital movement processing module) built in the MPU-9400, it is only necessary to convert a body posture quaternion parameter output by the sensor hardware into an Eulerian angle, to obtain a posture angle of the body in a triaxial direction, substantially save operation resources of hardware and improve robustness of the system. Meanwhile, the Eulerian angle obtained by solving is used to convert the original acceleration value in the triaxial direction to obtain a triaxial acceleration upon conversion from the body coordinate system to a geographical system. At this time, the triaxial angular speed and longitudinal perpendicular-to-ground acceleration generated by forearm movement during action implementation serve as real-time dynamic analysis basis and provide the user with a visual and accurate action effect. An output interface of the original data of the three-axis gyro, three-axis acceleration sensor and three-axis magnetic induction sensor and an output interface of fused data after DMP processing are I2C (two wire-type serial bus), and a data refreshing frequency may achieve 200Hz, and a higher real-time is provided for the movement control and identification.

The multi-path selection module 22 may employ a 74LS401 which is a one-out-of-eight multi-path selector. In the embodiment of the present invention, six channels are employed, a three-wire input terminal thereof is controlled via the processing module 12, and an output channel is selected. A switching delay of the multi-path selector module 22 is lower than 50ns, and may be neglected relative to human muscle movement. Hence, a multi-path switching solution is feasible.

The voltage-controlled constant-current source 23 may employ an LT1639, a maximum design of stimulation signal intensity is 20mA, a load is assumed as 1k Ω, and LT1639 is required to provide 20V voltage. Due to low-power consumption requirement and parameter requirements and circuit miniature, the present embodiment employs LT1639 four operational amplifier chip. The LT1639 uses a ± 40V working voltage. Due to miniature and low-power consumption requirements of the electromyographic stimulation, an LT1640 is selected to complete voltage conversion. The LT1640 is purposefully designed by Linear corporation for a high-power system having 350mA current limitation and a 1.2V-40V input voltage range, and adapted for LT1639 power supply. It can still operate when its input is reduced to 1V, and its output voltage reaches up to 34V

The communication unit 40 may employ an ESP8266 which is a complete and self-contained wifi network solution and substantially saves the size.

A training assistance method executed by the above training assistance apparatus according to the present invention is as shown in Fig. 6, and mainly comprises the following steps:
In step 601, an electromyographic signal of a user's specific position during implementation of actions is collected and amplified and filtered.

The step is executed by the collecting unit of the training assistance apparatus. For particulars, please refer to the depictions in the apparatus embodiments and detailed depictions will not be offered any more here.

In step 602, an optimal electromyographic signal is determined according to the amplified and filtered electromyographic signal, and a stimulation signal is formed by using the optimal electromyoelectrical signal.

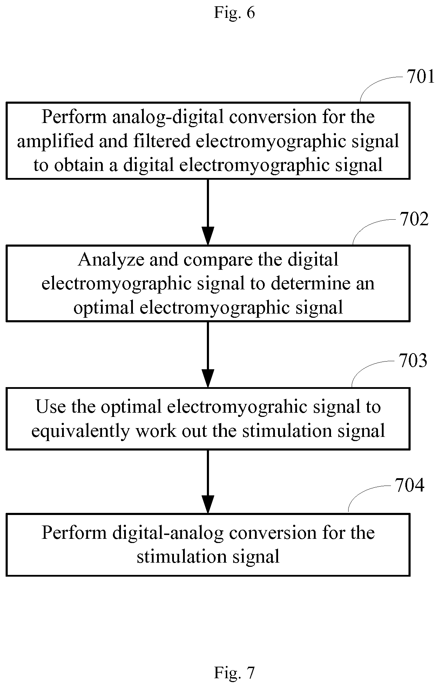

The step is executed by the main control unit of the training assistance apparatus. Specifically, the step may comprise the following sub-steps as shown in Fig. 7

Step 701: performing analog-digital conversion for the amplified and filtered electromyographic signal to obtain a digital electromyographic signal.

The step is executed by the ADC in the main control unit of the training assistance apparatus.

Step 702: analyzing and comparing the digital electromyographic signal to determine an optimal electromyographic signal.

The step is executed by the processing module of the main control unit. Specifically, the digital electromyographic signal may be analyzed to obtain various indices of the action of this time, for example indices such as correctness, tension, and rhythmical accuracy of the action. The specific indices may depend on a specific training program. The electromyographic signal of the action of this time is compared with the electromyographic signals of actions in the past in respect of various indices, to determine the optimal electromyograhic signal.

In addition, it is further feasible to determine the optimal electromyograhic signal in conjunction with movement data collected by the nine-axis sensor.

Step 703: using the optimal electromyograhic signal to equivalently work out the stimulation signal.

The step is also executed by the processing module in the main control unit, a cyclic signal matching with the optimal electromyographic signal is determined in a power equivalence manner, and the cyclic signal is amplified as the stimulation signal. Preferably, the frequency of the cyclic signal is over twice the electromyographic signal frequency.

The cyclic signal may be sinusoidal wave, square wave or the like. It is feasible to obtain an electromyograhic signal intensity corresponding to each sampling point at a fixed sampling frequency. Since a frequency of the stimulation signal might be inconsistent with the frequency of the electromyographic signal, preferably over twice the electromyographic signal frequency, it is feasible to emit the stimulation signal of more than two cycles at an interval time of each sampling point. The power of the stimulation signal is closely related to the amplitude, and the amplitude of the stimulation signal of more than two cycles is equivalently worked out according to the intensity of the optimal electromyographic signal of each cycle.

The amplifying multiple employed upon amplifying the cyclic signal satisfies V*G<R*I, wherein V is a maximum intensity of the optimal electromyographic signal, R is a human body equivalent resistance, and I is a stimulation current intensity that can be received by the human body without pains.

Step 704: performing digital-analog conversion for the stimulation signal.

The step is executed by the DAC module of the main control unit.

Further referring to Fig. 6, in step 603, the stimulation current is formed according to the stimulation signal, and the stimulation current acts upon the user's specific position.

The step is executed by the discharging unit of the electromyographic signal collecting apparatus. First, the stimulation signal is converted into a bipolar signal, and then the bipolar signal serves as an input signal of the voltage-controlled constant-current source to obtain the stimulation current output by the voltage-controlled constant-current source, and finally the discharging electrode applies the stimulation current to the user's specific position.

The above training assistance apparatus and training assistance method according to the present invention are adapted for training of musical instruments such as piano or guitar, and also adapted for training of sports such as golf and tennis.

In the embodiments of the present invention, it should be understood that the system, apparatus and method disclosed can be implemented in other ways. For example, the above-mentioned apparatus embodiments are only illustrative, e.g., the division of the units is merely logical one, and, in reality, they can be divided in other ways.

The units described as separate parts may be or may not be physically separated. One can select some or all of the units to achieve the purpose of the embodiment according to the actual needs.

Further, in the embodiments of the present invention, functional units can be integrated in one processing unit, or they can be separate physical presences; or two or more units can be integrated in one unit. The integrated unit described above can be realized as hardware, or they can be realized with hardware and software functional unit.

The aforementioned integrated unit in the form of software function units may be stored in a computer readable storage medium. The aforementioned software function units are stored in a storage medium, including several instructions to instruct a computer device (a personal computer, server, or network equipment, etc.) or processor to perform some steps of the method described in the various embodiments of the present invention. The aforementioned storage medium includes various media that may store program codes, such as U disk, removable hard disk, read-only memory (ROM), a random access memory (RAM), magnetic disk, or an optical disk.

What are described above are only preferred embodiments of the present disclosure, and not intended to limit the present disclosure. Any modifications, equivalent substitutes and improvements within the spirit and principles of the present disclosure all fall within the protection scope of the present invention.

## Claims

1. A training assistance apparatus, wherein the training assistance apparatus comprises: a collecting unit, a main control unit and a discharging unit;
the collecting unit is configured to collect an electromyographic signal of a user's specific position during implementation of actions and output it to the main control unit after amplification and filtering;
the main control unit is configured to determine an optimal electromyographic signal according to the received electromyogaphic signal, use the optimal electromyoelectrical signal to form a stimulation signal, output the stimulation signal to the discharging unit;
the discharging unit is configured to form a stimulation current according to the stimulation signal and apply the stimulation current upon the user's specific position.

2. The training assistance apparatus according to claim 1, wherein the collecting unit comprises a detection electrode, an amplifying module and a filtering module;
the detection electrode is disposed at the user's specific position and used to collect the electromyographic signal at the user's specific position, and output it to the amplifying module;
the amplifying module is configured to perform amplifying processing for the electromyoelectrical signal and then output it to the filtering module;
the filtering module is configured to perform filtering processing for the electromyographic signal output by the amplifying module, and then output to the main control unit.

3. The training assistance apparatus according to claim 1, wherein the main control unit comprises an analog-digital conversion module, a processing module and a digital-analog conversion module;
the analog-digital conversion module is configured to perform analog-digital conversion for the electromyographic signal output by the collecting unit to obtain a digital electromyographic signal and then output it to the processing module;
the processing module is configured to use the received digital electromyographic signal to determine an optimal electromyographic signal, use the optimal electromyogrphic signal to equivalently work out a simulation signal and output the stimulation signal to the digital-analog conversion module;
the digital-analog conversion module is configured to perform digital-analog conversion for the received stimulation signal and then output it to the discharging unit.

4. The training assistance apparatus according to claim 3, wherein the training assistance apparatus further comprises: a communication unit;
upon determining the optimal electromyographic signal, the processing module specifically executes the following: send the collected electromyographic signal to a terminal equipment via the communication unit, and obtain the stimulation signal returned by the terminal equipment via the communication unit, the stimulation signal being equivalently worked out using the optimal electromyographic signal after the terminal equipment analyzes and compares the collected electromyograhic signal to determine the optimal electromyographic signal; or, analyze and compare the collected electromyographic signal to determine the optimal electromyographic signal, and send the optimal electromyograpic signal to the terminal equipment via the communication unit, obtain the stimulation signal returned by the terminal equipment via the communication unit, the stimulation signal being equivalently worked out by the terminal equipment using the optimal electromyographic signal.

5. The training assistance apparatus according to claim 3 or 4, wherein upon determining the optimal electromyographic signal, the processing module specifically executes the following: analyze the received digital electromyographic signal to obtain various indices of the action of this time, compare the electromyographic signal of the action of this time with the electromyographic signals of actions in the past in respect of various indices, to determine the optimal electromyograhic signal therein.

6. The training assistance apparatus according to claim 3, wherein using the optimal electromyographic signal to equivalently work out the stimulation signal, the processing module specifically executes determining a cyclic signal matching with the optimal electromyographic signal in power, and amplify the cyclic signal as the stimulation signal.

7. The training assistance apparatus according to claim 6, wherein a frequency of the cyclic signal is over twice a frequency of the electromyographic signal.

8. The training assistance apparatus according to claim 6, wherein an amplifying multiple employed by the processing module upon amplifying the cyclic signal satisfies V*G<R*I, wherein the V is a maximum intensity of the optimal electromyographic signal, R is a human body equivalent resistance, and I is a stimulation current intensity that can be received by the human body without pains.

9. The training assistance apparatus according to claim 3, wherein the training assistance apparatus further comprises a nine-axis sensor;
the nine-axis sensor is used to collect the user's movement data and output it to the processing module;
the processing module is further used to combine with the movement data to determine the optimal electromyographic signal.

10. The training assistance apparatus according to claim 1, wherein the discharging unit comprises a forward side summator, a voltage-controlled constant-current source and a discharging electrode;
the forward side summator is configured to convert the stimulation signal output by the main control unit into a bipolar signal and then output the bipolar signal to the voltage-controlled constant-current source;
the voltage-controlled constant-current source is configured to output a corresponding stimulation current according to the input bipolar signal;
the discharging electrode is used to enable the stimulation current output by the voltage-controlled constant-current source to act upon the user's specific position.

11. The training assistance apparatus according to claim 10, wherein the collecting unit and the discharging unit respectively have N channels, wherein the N is a positive integer greater than 1, and the channel of the collecting unit corresponds one-to-one with the channel of the discharging unit;
the discharging unit further comprises: a multi-path selection module controlled by the main control unit to select a channel, and send the bipolar signal output by the forward side summator to the corresponding voltage-controlled constant-current source module of the corresponding channel in turn at divided time.

12. The training assistance apparatus according to claim 1, the detection electrode in the collecting unit and the discharging electrode in the discharging unit share a differential electrode in a frequency division manner.

13. A training assistance method, wherein the training assistance method comprises:
collecting an electromyographic signal of a user's specific position during implementation of actions and performing amplification and filtering for the electromyographic signal;
determining an optimal electromyographic signal according to the electromyogaphic signal after amplification and filtering, and using the optimal electromyoelectrical signal to form a stimulation signal;
forming a stimulation current according to the stimulation signal and applying the stimulation current upon the user's specific position.

14. The training assistance method according to claim 13, wherein the determining an optimal electromyographic signal according to the electromyogaphic signal after amplification and filtering, and using the optimal electromyoelectrical signal to form a stimulation signal comprises:
performing analog-digital conversion for the amplified and filtered electromyographic signal to obtain a digital electromyographic signal;
analyzing and comparing the digital electromyographic signal to determine an optimal electromyographic signal;
using the optimal electromyograhic signal to equivalently work out the stimulation signal;
performing digital-analog conversion for the stimulation signal.

15. The training assistance method according to claim 14, wherein the analyzing and comparing the digital electromyographic signal to determine an optimal electromyographic signal comprises:
analyzing the digital electromyographic signal to obtain various indices of the action of this time;
comparing the electromyographic signal of the action of this time with the electromyographic signals of actions in the past in respect of various indices, to determine the optimal electromyograhic signal.

16. The training assistance method according to claim 14, wherein the using the optimal electromyograhic signal to equivalently work out the stimulation signal comprises:
determining a cyclic signal matching with the optimal electromyographic signal power, and amplifying the cyclic signal as the stimulation signal.

17. The training assistance method according to claim 16, wherein a frequency of the cyclic signal is over twice a frequency of the electromyographic signal.

18. The training assistance method according to claim 16, wherein an amplifying multiple employed upon amplifying the cyclic signal satisfies V*G<R*I, wherein the V is a maximum intensity of the optimal electromyographic signal, R is a human body equivalent resistance, and I is a stimulation current intensity that can be received by the human body without pains.

19. The training assistance method according to claim 13, wherein the method further comprises: using a nine-axis sensor to collect the user's movement data, and further combining the movement data upon determining the optimal electromyographic signal.

20. The training assistance method according to claim 13, wherein the forming a stimulation current according to the stimulation signal comprises:
converting the stimulation signal into a bipolar signal;
using the bipolar signal as an input signal of the voltage-controlled constant-current source to obtain the stimulation current output by the voltage-controlled constant-current source.
